# EUROPEAN PATENT APPLICATION

(11) **EP 1 764 115 A1**
(43) Date of publication of application: **21.03.2007**
(21) Application number: 05255724.6
(22) Date of filing: 15.09.2005
(51) Int. Cl.: A61L 2/20

(54) **Sterilization method**

(71) Applicant: SHIBUYA KOGYO CO., LTD., Kanazawa-Shi, Ishikawa-ken (JP)
(72) Inventor: Imai, Kunihiro, c/o Shibuya Kogyo Co. Ltd., Kanazawa-Shi, Ishikawa-Ken (JP); Watanabe, Souma, c/o Shibuya Kogyo Co. Ltd., Kanazawa-Shi, Ishikawa-Ken (JP)
(74) Representative: Naylor, Matthew John

(57) **Abstract**

After making the relative humidity in a working chamber 1 to 10% or less beforehand, hydrogen peroxide vapor supply means 2 is operated, and hydrogen peroxide solution is dropped from an injector 14 into an evaporator 12 for 3 minutes at a flow rate of 5 g/min. Thereby, hydrogen peroxide vapor is rapidly supplied to the above-mentioned working chamber 1, and the concentration of the hydrogen peroxide vapor in the working chamber 1 is set to about 1300 ppm.

Dropping of hydrogen peroxide solution with the injector 14 is stopped and the state of a closed circuit between the working chamber and the hydrogen peroxide vapor supply means is maintained for a predetermined time, and if the concentration of hydrogen peroxide vapor becomes the predetermined concentration or below, ventilation means 4 is operated to remove the hydrogen peroxide vapor in the working chamber, and sterilization is terminated.

Sterilization method in which sterilization can be carried out in a shorter time and at a lower cost than in conventional methods is provided.

## Description

### Field of the invention

The present invention relates to a sterilization method, and more specifically to a sterilization method for sterilizing the inside of a closed working chamber with hydrogen peroxide vapor.

### Description of the Prior Art

Conventionally, in order to fill a vial, etc. with a chemical agent and perform culturing of microbes, etc., a working chamber isolated from the external atmosphere and the inside of which is sterilized has been used, and it is necessary to sterilize the inside of the above-mentioned working chamber beforehand in order to do these operations.

As a method for sterilizing such a working chamber, there is known a method of performing sterilization by filling the working chamber with hydrogen peroxide vapor, and specifically the following sterilization methods are known.

First, there is known a sterilization method by determining the saturation factor from the concentration of hydrogen peroxide vapor supplied to the working chamber and the dew point concentration of hydrogen peroxide vapor according to the temperature and humidity in the working chamber, supplying hydrogen peroxide vapor while maintaining the concentration at a constant level, and further adjusting the temperature and humidity in the working chamber so that the above-mentioned saturation factor may be close to 100% thereby performing sterilization (Patent Document 1).

Next, there is known a sterilization method by condensing the hydrogen peroxide vapor in a saturated condition and allowing it to deposit on the surface of the working chamber and utilizing this condensing property to perform sterilization, wherein the concentration of the vapor in the working chamber is maintained at a saturated vapor level so that the condensation of the above-mentioned hydrogen peroxide vapor may continue (Patent Document 2).

[Patent Document 1] WO 97/47331

[Patent Document 2] WO 01/70282

In the above-mentioned Patent Documents 1 and 2, however, in order to perform sufficient sterilization, supply of hydrogen peroxide vapor should be continued for a considerable time even after the concentration of the hydrogen peroxide vapor in the working chamber reached a constant concentration, and a long time was needed before completing sterilization.

In addition, in the above-mentioned Patent Documents 1 and 2, it is necessary to measure the humidity, etc. in the working chamber all the time and to control the supply of hydrogen peroxide vapor according to the measured results in order to maintain the above-mentioned saturation factor and saturated vapor level, a sensor and control means therefor are needed, which incurs high cost.

### Summary of the Invention

In consideration of these problems, the present invention provides a sterilization method in which sterilization can be carried out in a shorter time and at a lower cost than in conventional methods.

That is, the sterilization method of the present invention supplies hydrogen peroxide vapor into a closed working chamber to sterilize the working chamber, characterized by comprising:
setting beforehand an amount of hydrogen peroxide solution which can generate hydrogen peroxide vapor equal to or more than an amount to saturate almost all areas in the working chamber; vaporizing the set amount of hydrogen peroxide solution and rapidly filling the working chamber with hydrogen peroxide vapor; and subsequently maintaining the closed state of the working chamber for a required period of time.

According to the above-mentioned invention, since hydrogen peroxide vapor more than an amount to saturate almost all areas in the working chamber is generated and the working chamber is rapidly filled with this, the concentration of hydrogen peroxide vapor in the working chamber can be made higher compared with conventional sterilization methods.

And since it is known that the higher sterilization effect can be attained if the concentration of hydrogen peroxide vapor in the working chamber is made higher, it becomes possible to complete sterilization in a shorter time than before.

Furthermore, since it is sufficient just to supply the hydrogen peroxide vapor in an amount set beforehand without equipping the working chamber with a sensor and the like, such a sensor and the like as in the above-mentioned Patent Documents are not needed, and it becomes possible to sterilize at a lower cost that much.

### Brief Description of the Drawings

Fig. 1 is a plan view showing a working chamber and a hydrogen peroxide vapor supply means of a working example of the present invention;
Fig. 2 is a graph showing a sterilization method of a working example of the present invention; and
Fig. 3 is a graph showing a sterilization method different from the above-mentioned working example.

### Detailed Description of the Embodiments

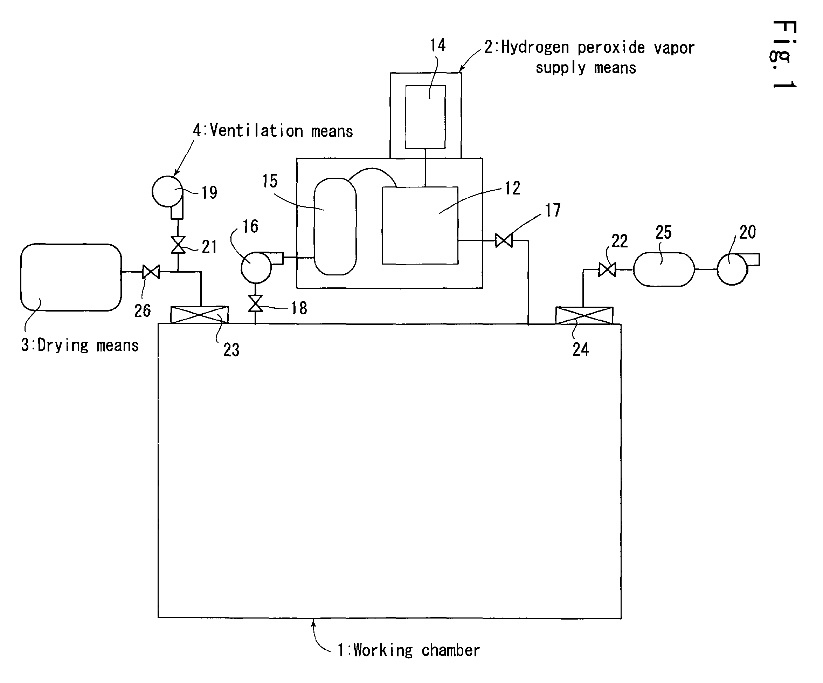

Hereinafter explaining illustrated Example, Fig. 1 shows a working chamber 1 for performing filling with a chemical agent, culturing of microbes, etc. under sterilized condition, hydrogen peroxide vapor supply means 2 to supply hydrogen peroxide vapor to the working chamber 1, drying means 3 to dry the inside of the working chamber 1, and ventilation means 4 to perform ventilation of the working chamber 1.

And the above-mentioned hydrogen peroxide vapor supply means 2, the drying means 3, and the ventilation means 4 are controlled by a control device which is not shown in the drawing, and sterilize the working chamber 1 automatically.

A conventionally known isolator is used for the above-mentioned working chamber 1 and the inside thereof is isolated from the external atmosphere, and in case of performing operations such as filling with a chemical agent as mentioned above, it is allowed to be positive in pressure by sterilized air supplied from a sterile air supply equipment which is not shown in the drawing, and maintained in a sterile condition.

The above-mentioned hydrogen peroxide vapor supply means 2 is equipped with an evaporator 12 which evaporates hydrogen peroxide solution, an injector 14 which drops hydrogen peroxide solution into this evaporator 12, and a blower 16 which supplies a overheated gas by the evaporator 12 through a heater 15 while suctioning the gas in the working chamber 1.

In addition, a first electromagnetic valve 17 is provided between the above-mentioned evaporator 12 and the working chamber 1, and a second electromagnetic valve 18 between the above-mentioned blower 16 and the working chamber 1, respectively and these first and second electromagnetic valves 17 and 18 are automatically opened and closed by a control device.

In order to evaporate the hydrogen peroxide solution dropped from the injector 14, the above-mentioned evaporator 12 is equipped with a metal evaporating plate which is not shown in the drawing, and surface temperature of this evaporating plate is heated at an arbitrary temperature by a control device and when hydrogen peroxide solution is dropped on this evaporating plate, the hydrogen peroxide solution evaporates and is vaporized in an instant.

Further, the above-mentioned injector 14 is a syringe pump to drop hydrogen peroxide solution of a predetermined concentration, and drops onto an evaporator 12 the whole amount of the hydrogen peroxide solution of an amount preset in the above-mentioned control device in a preset time. In this Example, 35% hydrogen peroxide solution commonly commercially available is used.

Furthermore, the above-mentioned heater 15 heats the gas suctioned by the above-mentioned blower 16 from the working chamber 1 and is configured to be able to heat gas and hydrogen peroxide vapor suctioned from the working chamber 1 to a predetermined temperature under control of a control device.

And while the hydrogen peroxide vapor supply means 2 is operated, the hydrogen peroxide vapor which flows into the working chamber 1 from the evaporator 12 is suctioned by the blower 16 and sent again to the heater 15 by opening the first and second electromagnetic valves 17 and 18, a closed circuit which maintains a closed state between the working chamber 1 and the hydrogen peroxide vapor supply means 2 is formed.

The above-mentioned ventilation means 4 is equipped with a suctioning blower 19 for supplying gas from the outside into the working chamber 1, and an evacuating blower 20 for discharging the gas in the working chamber 1 to the outside and third and fourth electromagnetic valves 21 and 22 are provided between the suctioning blower 19 and evacuating blower 20 and the working chambers 1, respectively.

HEPA filters 23 and 24 are also provided respectively at the positions at which the above-mentioned working chamber 1 communicates with the above-mentioned suctioning blower 19 and evacuating blower 20, and further a catalyst 25 which decomposes and detoxificates hydrogen peroxide vapor is provided between the evacuating blower 20 and a fourth electromagnetic valve 22.

With this ventilation means 4, the inside of the working chamber 1 can be ventilated with sterile air by supplying sterile air into the working chamber 1 through the above-mentioned suctioning blower 19 and the HEPA filter 23 and further discharging the hydrogen peroxide vapor filled in the working chamber 1 through the evacuating blower 20.

Furthermore, since hydrogen peroxide vapor discharged from the working chamber 1 is decomposed by the catalyst 25 while ventilation proceeds, hydrogen peroxide vapor is not discharged to the outside of the working chamber 1.

And the above-mentioned drying means 3 is connected to the working chamber 1 through a fifth electromagnetic valve 26 and the above-mentioned HEPA filter 23, and dried air from the drying means 3 is supplied into the working chamber 1 after asepticized with the HEPA filter 23.

And the dried air supplied into the working chamber 1 is evacuated by the evacuating blower 20 in the above-mentioned ventilation means 4, and it is possible to decrease the humidity in the working chamber 1 to an arbitrary humidity by supplying dried air for a predetermined time.

Based on the above-mentioned configuration, a sterilization method of the present invention is described below.

First, while the working chamber 1 is closed and when a start sterilization operation is given to a control device, the control device opens the fourth and fifth electromagnetic valves 22 and 26 while operating the above-mentioned drying means 3 and evacuating blower 20.

Thereby dried air from the drying means 3 is asepticized with the HEPA filter 23 and supplied into the working chamber 1, and the gas in the working chamber 1 is discharged outside by the evacuating blower 20.

Since the microbes and the like in the working chamber 1 are caught with the HEPA filter 24 at this time, it is prevented that these microbes and the like flow out of the working chamber 1.

And when a necessary and sufficient time passes or when it is detected that the relative humidity in the working chamber 1 becomes 10% or less at the sensor which is not shown in the drawing, the control device stops the drying means 3 and evacuating blower 20 and closes the above-mentioned fourth and fifth electromagnetic valves 22 and 26.

Next, the control device operates the above-mentioned hydrogen peroxide vapor supply means 2 and supplies hydrogen peroxide vapor into the working chamber 1. Here, the above-mentioned heater 15 and the evaporator 12 are fully preheated beforehand at this time. In this Example, the evaporating plate of the above-mentioned evaporator 12 is preheated at 107°C as an example.

Next, the control device opens the first and second electromagnetic valves 17 and 18, and controls the above-mentioned injector 14 to drop hydrogen peroxide solution onto the evaporator 12, and further supplies the gas in the working chamber 1 to the evaporator 12 through the heater 15 by the above-mentioned blower 16.

In this Example, the above-mentioned injector 14 is controlled so that hydrogen peroxide solution is continuously dropped for 3 minutes at a flow rate of 5 g/min, and the dropped hydrogen peroxide solution changes to hydrogen peroxide vapor by flash evaporation in an evaporator 12 in an instant.

The working chamber 1 used in this Example is a small isolator whose volume is about 0. 8 m³ and it has been confirmed by preliminary experiment that when 35% hydrogen peroxide solution is evaporated at a rate of 5 g per minute for 3 minutes as mentioned above in total of 15 g, hydrogen peroxide vapor does saturate and condensation occurs not partly but almost over the whole area in the working chamber 1.

The thus evaporated hydrogen peroxide vapor is supplied into and fill the working chamber 1 along with the gas heated at the above-mentioned heater 15, and after that, the hydrogen peroxide vapor in this working chamber 1 is suctioned by the above-mentioned blower 16, and it is circulated in the closed circuit consisted of the working chamber 1 and the hydrogen peroxide vapor supply means 2.

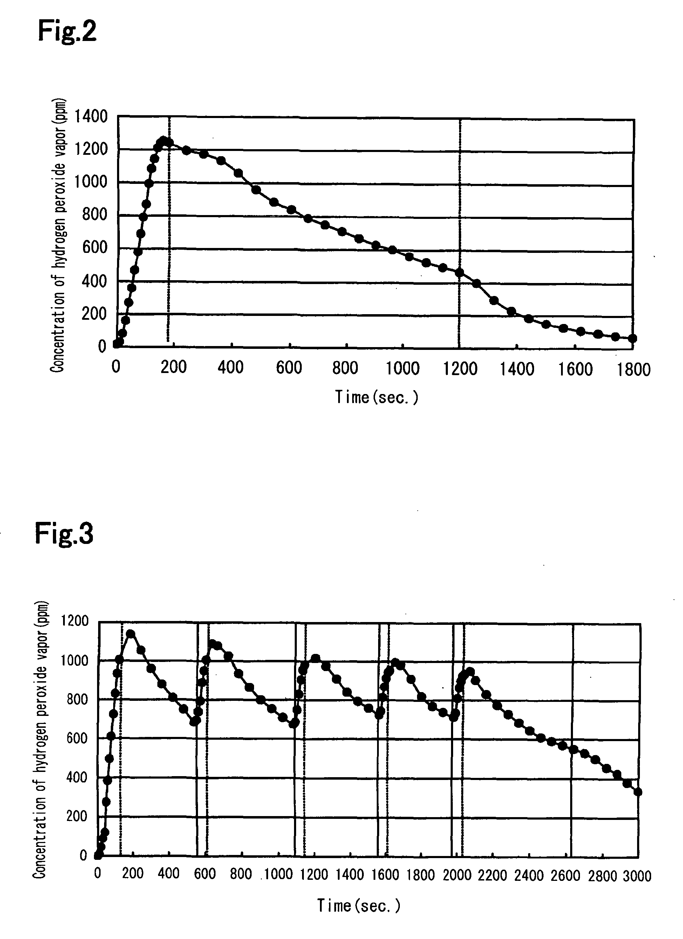

Fig. 2 is a graph which indicates the time from the start of operation of the hydrogen peroxide vapor supply means 2 to the end of ventilation by the ventilation means 4 on the horizontal axis, and shows the result of actually measured concentration of hydrogen peroxide vapor inside the working chamber 1 on the vertical axis.

As shown in this graph, it turns out that when the whole amount of 15 g of hydrogen peroxide solution is vaporized and rapidly made to fill the working chamber 1 of the volume of about 0.8 m³ for a short time of 3 minutes, the hydrogen peroxide vapor concentration in the space in the working chamber 1 rises rapidly to about 1300 ppm.

Conventionally, as for the hydrogen peroxide vapor concentration in the air in a normal state (chamber temperature, atmospheric pressure), about 700 ppm is supposed to the limit, but it has been discovered that it is possible to raise hydrogen peroxide vapor concentration even to a high concentration exceeding 1000 ppm by making the hydrogen peroxide vapor more than an amount by which saturation of almost all the areas of the working chamber 1 is confirmed to fill the working chamber 1 rapidly as in this Example.

It has been confirmed that when such a high-concentration state is attained, the inside of the working chamber 1 is saturated with hydrogen peroxide vapor over almost all the spaces in the working chamber 1, and the condensation occurs and it looks whitely and cloudy. However, it is not the object of the present invention to generate condensation but to use the property that the concentration continues to rise until condensation occurs on the whole of the space and hydrogen peroxide vapor concentration falls, and confirmation of generation of condensation throughout the space is used as an indication for determining the maximum point or peak of concentration.

As mentioned above, in this Example, an amount of the hydrogen peroxide solution to be used is set beforehand from the amount of vapor required to allow the concentration of the hydrogen peroxide vapor in working chamber 1 to be the concentration as mentioned above in accordance with conditions such as the volume and humidity, and temperature of the above-mentioned working chamber 1, and the dropping amount and dropping time of hydrogen peroxide solution by the injector 14 is determined.

And according to the result of this experiment, the dropping amount and dropping time are set in the control device, and in this Example, when the whole amount of the set amount has been evaporated at the time when 3 minutes has passed since the operation of the hydrogen peroxide vapor supply means 2 is started, dropping of hydrogen peroxide solution by the injector 14 is stopped.

In this way, when terminating dropping of the hydrogen peroxide solution from the injector 14 in the hydrogen peroxide vapor supply means 2, the control means continues to operate the above-mentioned heater 15 and blower 16 succeedingly while opening the first and second electromagnetic valves 17 and 18 and circulating hydrogen peroxide vapor between the working chamber 1 and hydrogen peroxide vapor supply means 2 maintained in a closed state.

When the dropping of the hydrogen peroxide solution from the injector 14 is terminated, no more additional hydrogen peroxide vapor is supplied, and already supplied hydrogen peroxide vapor is condensed and accordingly the concentration will gradually decrease.

Although this is considered to be attributable to formation of liquid droplets by condensation, absorption onto the surface of substances present in the working chamber 1, and natural decomposition of hydrogen peroxide itself, the decrease is gradual as compared with a rapid rise, and it turns out that the concentration exceeding 500 ppm effective for sterilization is maintained 800 seconds or more during the decreasing term. Since in the case of this Example effective concentration is maintained about 1000 seconds if the time for the rising term of concentration is included, it is judged to be enough to perform the sterilization in the working chamber 1.

In the present invention, attention is paid to this point and after rapidly filling in the working chamber 1 with hydrogen peroxide vapor and raising concentration, the closed state of the working chamber 1 is maintained for a required time, i.e., until it is less than the concentration effective for sterilization.

In order to confirm the effect of sterilization by this Example, sterilization was performed while a conventionally known biological-indicator using biological index was accommodated in the working chamber 1 and culturing the biological-indicator over a predetermined period was performed after that, and as a result, it has been confirmed that multiplication of the bacillus used as an index is not recognized, and that sterilization is effective.

Then, after a predetermined time passes (in 1200 seconds after starting the operation of the hydrogen peroxide vapor supply means 2 in Fig. 2), the control device stops the above-mentioned blower 16, closes the first and second electromagnetic valves 17 and 18 and stops the hydrogen peroxide vapor supply means 2.

When the hydrogen peroxide vapor supply means 2 is stopped, the control device operates the ventilation means 4 and operates the above-mentioned suctioning blower 19 and evacuating blower 20 and at the same time opens the third and fourth electromagnetic valves 21 and 22.

Thereby air sterilized by the suctioning blower 19 flows into the working chamber 1 through a HEPA filter 28, and the hydrogen peroxide vapor in the working chamber 1 is evacuated to the outside by evacuating blower 20.

Since hydrogen peroxide vapor is decomposed by the catalyst 25 at this time, hydrogen peroxide vapor is not discharged to the outside of the working chamber 1.

And if the predetermined time passes or the concentration of hydrogen peroxide vapor in the working chamber 1 decreases to a predetermined value by a sensor which is not shown in the drawing in the working chamber 1, the above-mentioned suctioning blower 19 and evacuating blower 20 are stopped and the third and fourth electromagnetic valves 21 and 22 are closed to terminate ventilation in the working chamber 1, and sterilization of the working chamber 1 is completed.

As described above, according to the sterilization method of this Example, high concentration with a concentration peak of about 1300 ppm can be obtained by vaporizing hydrogen peroxide solution of an amount set beforehand, and making it rapidly fill the working chamber 1 for a short period of time of 3 minutes in order to sterilize it.

And although the concentration of hydrogen peroxide vapor decreases effective concentration is still maintained even after the supply of hydrogen peroxide vapor is stopped, high sterilization effect can continue and the time from the start to the end of sterilization can be shorter than those in the above-mentioned Patent Documents 1 and 2.

And since what is necessary is just to preliminarily set an amount of the hydrogen peroxide solution to be used and to vaporize and supply this in whole amount, there is no necessity of controlling the injector 14 while detecting the concentration in the working chamber using a sensor etc. , and it becomes possible to suppress the cost for constructing the equipment by omitting an expensive sensor and a feedback control means.

In the meantime, it is needless to say that the dropping amount and dropping time of hydrogen peroxide solution by the injector 14 vary with volume of the working chamber 1, environment such as temperature and atmospheric pressure, or the ventilation capability of above-mentioned blower 16, and are not limited to the dropping amount and dropping time of the injector 14 described in this Example.

Specifically, it suffices that the concentration of hydrogen peroxide vapor be maintained 500 ppm or more for at least 800 seconds after the sterilization operation is started and average concentration be maintained by 800 ppm or more in order to obtain good sterilization effect.

And for that purpose, it is desirable that at least 10 g of 35% concentration hydrogen peroxide solution for a volume of 1 cubic meter of the working chamber 1 is used and vaporized and in addition the hydrogen peroxide solution of that amount is vaporized in 6 minutes at the longest.

This is because experimental results have revealed that even if the dropping time of hydrogen peroxide solution by the above-mentioned injector 14 is set up to be 6 minutes or more and hydrogen peroxide vapor is supplied, the inside of the working chamber is saturated and concentration would not go up above, and the condensation of hydrogen peroxide on the whole start at about 700 ppm.

And as a result of supplying hydrogen peroxide vapor in the working chamber 1 according to the above-mentioned conditions, it has been revealed that the concentration of hydrogen peroxide vapor in the working chamber 1 amounted to 800 ppm or more is enough although it may depend on the required level of sterilization, and more preferably, when the concentration of 1000 ppm or more was obtained, it is possible to obtain good sterilization effect.

In addition, since hydrogen peroxide vapor may be absorbed by the equipment installed in the working chamber 1, it is necessary to take these conditions into consideration and set the amount of the hydrogen peroxide solution set beforehand, and since it is known that the above-mentioned HEPA filter especially ready to absorb, it is necessary to take into consideration especially the existence and the number of installed HEPA filter.

That is, under conditions in which a little amount of hydrogen peroxide vapor is absorbed, the concentration decreasing rate after the peak is small and it is possible to maintain the effective concentration for a required period of time and a concentration peak of about 800 ppm is enough, but it is necessary to make a concentration peak high under conditions in which a lot amount is absorbed. Since this also influences the amount of the hydrogen peroxide solution to be used, it also needs to take these conditions into consideration and set the amount used.

And Fig. 3 shows a graph at the time of performing sterilization by a sterilization method different from the above-mentioned Example, and hydrogen peroxide vapor supplied into the working chamber 1 in two or more separate times in this Example.

Describing specific operation, in this Example, the hydrogen peroxide vapor supply means 2 is operated after the inside of the working chamber 1 is dried by the above-mentioned drying means 3 so that relative humidity may be 10% or less.

And in this Example, hydrogen peroxide solution is dropped from the injector 14 in five separate times to the evaporator 12, and hydrogen peroxide vapor is supplied by dropping at a rate of 5 g/min for 2 minutes for the first time, and by dropping at a rate of 5 g/min for 1 minute respectively for the second time and thereafter.

In addition, there are intervals of 5 minutes, respectively after dropping is completed at the first time to the fourth time, and a closed state is maintained by the working chamber 1 and the hydrogen peroxide vapor supply means 2, and the state of the above-mentioned closed circuit is now maintained for 10 minutes after dropping of the fifth time is completed.

After that, it is the same as that of the above-mentioned Example, and when the hydrogen peroxide vapor supply means 2 is stopped, the ventilation means 4 is operated, the hydrogen peroxide vapor in working chamber 1 is evacuated, and sterilization operation is terminated.

This shows that although it was able to determine the times of supply by confirming the degree of sterilization using a biological indicator and consequently one hydrogen peroxide vapor supply is enough in the case of the Example shown above, if required sterilization effect is not obtained, hydrogen peroxide vapor may be supplied similarly again to raise the concentration, and effective concentration can be maintained for a required period of time.

That is, in the case of condition where decrease in the concentration from the peak is rapid, supply of hydrogen peroxide vapor may be repeated and the number of times thereof can be determined by confirming the degree of sterilization beforehand.

By supplying hydrogen peroxide vapor rapidly into the working chamber 1 as in the above-mentioned Example, the concentration of hydrogen peroxide vapor in the working chamber 1 becomes high, and high sterilization efficiency can be obtained in the case of this Example.

However, as shown in Fig. 3, the peak of concentration becomes lower and the validity per amount of the hydrogen peroxide solution used also becomes lower as generation of hydrogen peroxide vapor is repeated, and accordingly it is desirable to limit the time to about three times at most.

Although the above-mentioned Examples describe sterilization of a working chamber, it is needless to say that the present invention can be used also for uses such as sterilization, disinfection and decontamination in addition to this.

In addition, the above-mentioned first and second electromagnetic valves 17 and 18 may be closed to completely close the inside of the working chamber 1 during the period in which the closed state of the above-mentioned working chamber 1 is maintained in the above-mentioned Examples.

When such a sterilization method is adopted, it is possible to make the construction of the above-mentioned hydrogen peroxide vapor supply means 2 changed into a so-called flow type hydrogen peroxide evaporation supply means in which the hydrogen peroxide vapor in the working chamber 1 is not circulated by blower 16.

And as for the above-mentioned ventilation means 4, it is also possible not to adopt ventilation means equipped with a suctioning blower 19 and an evacuating blower 20 as in the above-mentioned Examples but adopt so-called circulation type ventilation means.

Furthermore, when the volume of the working chamber 1 is large, it is also possible to install two or more of the above-mentioned hydrogen peroxide vapor supply means 2.

## Claims

1. A sterilization method which supplies hydrogen peroxide vapor into a closed working chamber to sterilize the working chamber, **characterized by** comprising:
setting beforehand an amount of hydrogen peroxide solution which can generate hydrogen peroxide vapor equal to or more than an amount to saturate almost all areas in the working chamber; vaporizing the set amount of hydrogen peroxide solution and rapidly filling the working chamber with hydrogen peroxide vapor; and subsequently maintaining the closed state of the working chamber for a required period of time.

2. The sterilization method according to claim 1, **characterized in that** the amount of hydrogen peroxide solution to be vaporized is set so that at least 10 g of 35% concentration hydrogen peroxide solution is present per cubic meter of the working chamber.

3. The sterilization method according to claim 1 or 2, **characterized in that** the supply time of hydrogen peroxide vapor does not exceed 6 minutes.

4. The sterilization method according to any one of claims 1 to 3, **characterized in that** the hydrogen peroxide vapor concentration in the working chamber is raised to 800 ppm or more by filling the working chamber rapidly with hydrogen peroxide vapor.

5. The sterilization method according to any one of claims 1 to 4, **characterized in that** after the working chamber is rapidly filled with hydrogen peroxide vapor by the set amount of hydrogen peroxide solution, hydrogen peroxide solution is vaporized and the working chamber is rapidly filled with vapor again.

6. The sterilization method according to any one of claims 1 to 5, **characterized in that** the number of supplying times of hydrogen peroxide vapor is determined by confirming the degree of sterilization.
